# FASCICULE DE BREVET EUROPEEN

(11) **EP 2 723 724 B1**
(45) Date de publication et mention de la délivrance du brevet: **30.11.2016**
(21) Numéro de dépôt: 12730904.5
(22) Date de dépôt: 22.06.2012
(51) Int. Cl.: C07D 295/088, C07D 295/096, C07D 213/74, C07D 241/20, A61K 31/495, A61P 3/10

(54) **DÉRIVÉS DE PIPÉRAZINE, LEURS PROCÉDÉS DE PRÉPARATION ET LEURS UTILISATIONS DANS LE TRAITEMENT DE L'INSULINORÉSISTANCE**
PIPERAZIN-DERIVATE, DEREN HERSTELLUNGSVERFAHREN UND DEREN VERWENDUNGEN IN DER BEHANDLUNG VON INSULINORESISTENZ
PIPERAZINE DERIVATIVES, THEIR PREPARATION PROCESSES AND THEIR USES IN THE TREATMENT OF INSULINORESISTANCE

(30) Priorité: 23.06.2011 FR 1155545
(43) Date de publication de la demande: 30.04.2014
(73) Titulaire: Metabolys, 69372 Lyon Cedex 08 (FR)
(72) Inventeur: MOINET, Gérard, F-91400 Orsay (FR); BAVEREL, Gabriel, F-69450 Saint Cyr Au Mont D'or (FR); NAZARET, Rémi, F-01000 Bourg En Bresse (FR); FERRIER, Bernard, F-69007 Lyon (FR)
(74) Mandataire: Lavoix
(86) Numéro de dépôt international: PCT/EP2012/062143
(87) Numéro de publication internationale: WO 2012/175707

(56) Documents cités:
- WO-A1-00/06558
- WO-A1-03/018553

## Description

La présente invention concerne de nouveaux composés utiles notamment dans le traitement de pathologies associées au syndrome d'insulinorésistance (ou syndrome X), notamment dans le traitement du diabète de type (II).

On observe depuis quelques années une augmentation importante des cas de diabète dans le monde. Actuellement, on compte environ 250 millions de diabétiques dans le monde et les prévisions pour 2030 sont de l'ordre de plus de 400 millions de diabétiques. Le diabète de type (I) (destruction des cellules produisant de l'insuline) est principalement traité par l'injection d'insuline. Le diabète de type (II), qui est le plus répandu (90% des cas de diabète), se caractérise par une résistance des tissus face à l'insuline et nécessite un traitement particulier.

De nombreux composés ont été proposés pour le traitement du diabète, notamment du diabète de type (II). On connaît notamment de FR2781797 et WO00/06558 des dérivés de pipérazine. On connaît également de WO02/100341 des dérivés phénylés.

Actuellement, dans environ 40% des cas, le traitement n'est pas efficace et le seuil de glycémie à jeun souhaité de 1,26 g/litre de sang n'est pas atteint.

II est donc nécessaire de proposer de nouveaux composés permettant un traitement plus efficace des pathologies associées au syndrome d'insulinorésistance.

Un objectif de la présente invention est donc de fournir des composés efficaces dans le traitement des pathologies associées au syndrome d'insulinorésistance.

Un autre objectif de la présente invention est de fournir un procédé de préparation de ces composés.

Un autre objectif encore de la présente invention est de fournir un moyen de traitement de pathologies associées au syndrome d'insulinorésistance, notamment diabète de type (II). Un objectif de la présente invention est de proposer des composés permettant notamment d'inhiber la néoglucogenèse.

D'autres objectifs encore apparaîtront à la lecture de la description de l'invention qui suit.

La présente invention concerne des composés de formule (I) 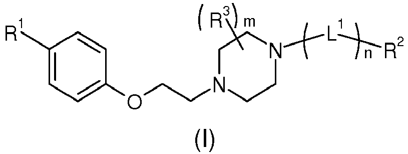 dans laquelle n et m représentent 0 , leurs énantiomères, diastéréoisomères et leurs sels d'addition avec des bases ou des acides pharmaceutiquement acceptables.

Dans les composés de formule (I) selon l'invention :
▪ R¹ représente un groupe -(CH₂)₄C(O)OH ;
▪ m représente 0 ;
▪ n représente 0 ;
▪ R² représente :
   - un groupe phényle, non substitué ou substitué par un ou plusieurs substituants, identiques ou différents, notamment choisi parmi :
      - un groupe alkoxy en C₁ à C₆, de préférence en C₁ à C₃, linéaire ou ramifié, par exemple méthoxy, éthoxy, propoxy, iso-propoxy, butoxy, ter-butoxy ;
      - un atome d'halogène, par exemple fluor, chlore, brome ;
      - un groupe hydrocarboné en C₁ à C₅, de préférence en C₁ à C₄, linéaire ou ramifié, par exemple méthyle, éthyle, propyle, butyle, iso-propyle, ter-butyle ;
      - un groupe hydrocarboné en C₁ à C₅, de préférence en C₁ à C₄, linéaire ou ramifié, substitué notamment par un ou plusieurs atomes d'halogène, par exemple trifluorométhyl ;
   - un groupe hétérocycle aromatique en 5 ou 6 membres, comprenant 1, 2 ou 3 hétéroatomes, identiques ou différents, choisis parmi l'azote, le soufre et l'oxygène;

De façon préférée, dans le composé de formule (I), R² représente :
- un phényl, non substitué ou substitué par un ou plusieurs substituants, identiques ou différents, notamment choisi parmi :
   - un groupe alkoxy en C₁ à C₆, de préférence en C₁ à C₃, linéaire ou ramifié, par exemple méthoxy, éthoxy, propoxy, iso-propoxy, butoxy, ter-butoxy ;
   - un atome d'halogène, par exemple fluor, chlore, brome ;
   - un groupe alkyle en C₁ à C₅, de préférence en C₁ à C₄, linéaire ou ramifié, substitué notamment par un ou plusieurs atomes d'halogène, par exemple trifluorométhyl ;
   de préférence le ou les substituants sont en position ortho ou para sur le phényl ;
- un hétéroaryl monocyclique choisi parmi 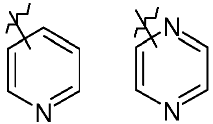 de préférence l'hétéroaryl est choisi parmi

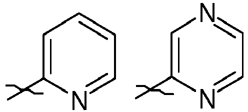

Les groupes hétérocycles aromatiques en 5 ou 6 membres sont par exemple choisis parmi

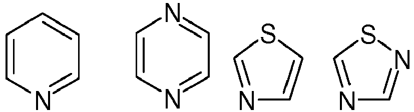

L'invention se rapporte également aux solvates des composés de formule (I). Les composés de formule (I) possèdent une fonction carboxylique et peuvent être salifiés. Ils peuvent alors se présenter sous la forme de sels d'addition avec des bases organiques ou minérales. Les sels d'addition avec des bases sont par exemple des sels pharmaceutiquement acceptables tels que les sels de sodium, les sels de potassium, les sels de calcium, lesquels sont obtenus en utilisant des hydroxydes de métaux alcalins et alcalino-terreux correspondants comme bases. Comme autre type de sels d'addition avec des bases pharmaceutiquement acceptables, on peut citer les sels avec des amines et notamment la glucamine, le N-méthylglucamine, le N,N-diméthylglucamine, l'éthanolamine, la morpholine, la N-méthylmorpholine ou la lysine.

Les composés de formule (I) peuvent également être salifiés avec des acides minéraux ou organiques et de préférence des acides pharmaceutiquement acceptables tels que les acides chlorhydrique, phosphorique, fumarique, citrique, oxalique, sulfurique, ascorbique, tartrique, maléique, mandélique, méthanesulfonique, lactobionique, gluconique, glucarique, succinique, sulfonique ou hydroxypropanesulfonique.

La présente invention concerne également un premier procédé (P1) de préparation des composés de formule (I) comprenant :
(a) la protection de la fonction acide (portée par R¹) d'un composé de formule (II) 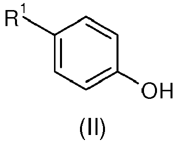 dans laquelle R¹ a la définition donnée pour la formule (I) ;
(b) la réaction du composé obtenu à l'étape (a) avec un composé de formule R⁴-(CH₂)₂-R⁵ dans laquelle R⁴ et R⁵, identiques ou différents représentent un groupe partant, de préférence R⁵ est meilleur nucléofuge que R⁴ ;
(c) la réaction du composé obtenu à l'étape (b) avec un composé de formule (III) 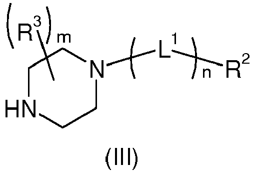 dans laquelle n, m et R ont les définitions données pour la formule (I) ;
(d) déprotection du composé obtenu à l'étape (c).

Les produits de départ sont commerciaux ou peuvent être facilement préparés par l'homme du métier sur la base de ses connaissances générales en chimie organique.

L'étape (a), correspondant à la protection de la fonction acide, peut se faire de toute manière connue par l'homme du métier, pourvue que la protection soit sélective de la fonction acide par rapport à la fonction alcool. Parmi les groupes protecteurs de la fonction carboxylique, ceux généralement décrits dans Protective Groups in Organic Synthesis, Greene T.W. et Wuts P.G.M, ed. John Wiley and Sons, 1991 et dans Protective Groups, Kocienski P.J., 1994, Georg Thieme Verlag, peuvent convenir. A titre d'exemple on peut envisager la protection de la fonction carboxylique sous la forme d'ester (alkyle en C₁-C₆, par exemple méthyle). Par exemple l'étape (a) peut se faire par réaction du composé de formule (I) avec le méthanol en présence d'un acide, notamment l'acide sulfurique.

L'étape (b) est de préférence mise en oeuvre dans un solvant polaire aprotique tel que l'acétonitrile, le diméthylformamide (DMF), l'acétone, le diméthylsulfoxide et les hydrocarbures halogénés tels que le dichlorométhane ou le dichloroéthane, à une température adaptée, notamment comprise entre 15 et 80°C, de préférence entre 15 et 35°C. Un solvant préféré est notamment l'acétonitrile. De façon avantageuse, cette étape a lieu en présence d'une base telle que le carbonate de potassium. L'homme du métier sait qu'un groupe partant est d'autant plus labile que l'espèce anionique correspondante est stable. Ainsi, R⁵ est plus nucléofuge que R⁴ correspond au fait que R⁵⁻ est plus stable que R⁴⁻. Les groupes partants R⁴ et R⁵, identiques ou différents, sont choisis parmi les atomes d'halogène, de préférence chlore et brome ; les groupes (C₆-C₁₀)arylsulfonyloxy, le groupe aryle étant éventuellement substitué par un ou plusieurs groupes alkyle en C₁ à C₆ ; les groupes (C₁-C₆)alkylsulfonyloxy dans lequel le groupe alkyle est éventuellement substitué par un ou plusieurs atomes d'halogène. De préférence R⁴ représente le chlore et R⁵ représente le brome.

L'étape (c) est une étape de substitution nucléophile pour laquelle les conditions opératoires pourront être facilement déterminées par l'homme du métier. Avantageusement, la réaction est mise en oeuvre dans un solvant polaire aprotique en présence d'une base. Des exemples de solvant appropriés sont l'acétonitrile, le diméthylformamide, l'acétone, le diméthylsulfoxide et les hydrocarbures halogénés tels que le dichlorométhane ou le dichloroéthane. A titre de base on peut citer les carbonates de métal alcalin ou alcalino-terreux, par exemple le carbonate de potassium. De façon avantageuse, la réaction de l'étape (c) est conduite à une température de 50 à 120°C, par exemple au reflux du solvant en présence d'un iodure de métal alcalin tel que l'iodure de potassium. La quantité d'iodure alcalin peut être variable et dépend essentiellement de la nature des réactifs, de la nature du solvant et de la température réactionnelle. La réaction est généralement stoechiométrique, il est néanmoins possible de travailler avec un léger excès de l'un ou de l'autre des réactifs.

L'étape de déprotection (d) peut être toute déprotection connue de l'homme du métier et compatible avec la protection mise en oeuvre à l'étape (a) permettant de récupérer la fonction acide. Par exemple, il peut s'agir d'une saponification en milieu basique, acide ou catalytique (Pd/C).

L'invention concerne également un second procédé (P2) de préparation d'un composé de formule (I) comprenant :
(i) la réaction d'un composé de formule (III) avec un composé de formule R⁴-(CH₂)₂-R⁵, dans laquelle R⁴ et R⁵ sont tels que définis dans le procédé (P1);
(ii) la protection de la fonction acide d'un composé de formule (II) ;
(iii) la réaction entre le composé obtenu à l'étape (i) et le composé obtenu à l'étape (ii) ;
(iv) la déprotection du composé obtenu à l'étape (iii).

Les produits de départ sont commerciaux ou peuvent être facilement préparés par l'homme du métier sur la base de ses connaissances générales en chimie organique.

L'étape (i) est de préférence mise en oeuvre dans un solvant polaire aprotique tel que l'acétonitrile, le diméthylformamide (DMF), l'acétone, le diméthylsulfoxide et les hydrocarbures halogénés tels que le dichlorométhane ou le dichloroéthane, à une température adaptée, notamment comprise entre 15 et 80°C, de préférence entre 15 et 35°C. Un solvant préféré est notamment l'acétonitrile. De façon avantageuse cette étape a lieu en présence d'une base telle que le carbonate de potassium.
L'étape (ii), correspondant à la protection de la fonction acide, peut se faire de toute manière connue par l'homme du métier, pourvue que la protection soit sélective de la fonction acide par rapport à la fonction alcool. Parmi les groupes protecteurs de la fonction carboxylique, ceux généralement décrits dans Protective Groups in Organic Synthesis, Greene T.W. et Wuts P.G.M, ed. John Wiley and Sons, 1991 et dans Protective Groups, Kocienski P.J., 1994, Georg Thieme Verlag, peuvent convenir. A titre d'exemple on peut envisager la protection de la fonction carboxylique sous la forme d'ester (alkyle en C₁-C₆, par exemple méthyle). Par exemple l'étape (a) peut se faire par réaction du composé de formule (I) avec le méthanol en présence d'un acide, notamment l'acide sulfurique.
L'étape (iii) est une étape de substitution nucléophile pour laquelle les conditions opératoires pourront être facilement déterminées par l'homme du métier. Avantageusement, la réaction est mise en oeuvre dans un solvant polaire aprotique en présence d'une base. Des exemples de solvant appropriés sont l'acétonitrile, le diméthylformamide, l'acétone, le diméthylsulfoxide et les hydrocarbures halogénés tels que le dichlorométhane ou le dichloroéthane. A titre de base on peut citer les carbonates de métal alcalin ou alcalino-terreux, par exemple le carbonate de potassium. De façon avantageuse, la réaction de l'étape (c) est conduite à une température de 50 à 120°C, par exemple au reflux du solvant en présence d'un iodure de métal alcalin tel que l'iodure de potassium. La quantité d'iodure alcalin peut être variable et dépend essentiellement de la nature des réactifs, de la nature du solvant et de la température réactionnelle. La réaction est généralement stoechiométrique, il est néanmoins possible de travailler avec un léger excès de l'un ou de l'autre des réactifs. L'étape de déprotection (iv) peut être toute déprotection connue de l'homme du métier et compatible avec la protection mise en oeuvre à l'étape (ii) permettant de récupérer la fonction acide. Par exemple, il peut s'agir d'une saponification en milieu basique, acide ou catalytique (Pd/C).

Les composés de la présente invention sont utiles comme médicament. Ils sont notamment utiles pour le traitement de pathologies associées au syndrome d'insulino-résistance (ou syndrome X), notamment du diabète de type II.
L'invention concerne également l'utilisation des composés selon l'invention en combinaison avec d'autres traitements, notamment d'autres traitements anti-diabétique.
De façon avantageuse, les composés de l'invention présentent une forte activité d'inhibition de la néoglucogenèse.

De façon avantageuse, les composés de l'invention présentent une activité de consommation du glucose.
L'invention concerne également l'utilisation d'un composé selon l'invention pour la préparation d'un médicament.
L'invention concerne également l'utilisation d'un composé selon l'invention pour la préparation d'un médicament pour le traitement de pathologies associées au syndrome d'insulino-résistance (ou syndrome X), notamment du diabète de type II.
L'invention concerne également l'utilisation des composés selon l'invention en combinaison avec d'autres traitements, notamment au moins un autre agent anti-diabétique, par exemple choisi parmi l'insuline, les sulfonyl-urée (par exemple glibenclamide, glimepiride, glipizide), les thiazoline-diones, les glinides, le DPP-IV inhibiteur (inhibiteur de dipeptidylpeptidase), le GLP-1 (Glucagon-like peptide-1) ou ses analogues.

La présente invention concerne également des compositions pharmaceutiques comprenant à titre de principe actif au moins un composé selon l'invention. Ces compositions comprennent également un véhicule ou excipient pharmaceutiquement acceptable.
Les compositions pharmaceutiques peuvent être sous toutes formes connues par l'homme du métier, notamment sous des formes destinées à l'administration par voie parentérale, orale, rectale, permuqueuse ou percutanée, de préférence par voie orale.
Les compositions selon l'invention seront présentées sous forme de solutés ou de suspensions injectables ou flacons multi-doses, sous forme de comprimés nus ou enrobés, de dragées, de capsules, de gélules, de pilules, de cachets, de poudres, de suppositoires ou de capsules rectales, de solutions de ou suspensions, pour l'usage percutané, dans un solvant polaire ou l'usage permuqueux.
Les excipients qui conviennent pour de telles administrations sont les dérivés de la cellulose ou la cellulose microcristalline, les carbonates alcalino-terreux, le phosphate de magnésium, les amidons, les amidons modifiés, le lactose pour des formes solides.
Pour l'usage rectal, le beurre de cacao ou les stéarates de polyéthylène glycol sont les excipients préférés.
Pour l'usage parentéral, l'eau, les solutés aqueux, le sérum physiologique, les solutés isotoniques sont les véhicules les plus commodément utilisés.
La posologie peut varier dans des limites importantes en fonction de l'indication thérapeutique, et de la voie d'administration, ainsi que de l'âge et du poids du sujet.
L'invention concerne également une composition comprenant à titre de principe actif au moins un composé selon l'invention en association avec au moins un autre agent anti-diabétique, notamment choisi parmi l'insuline, les sulfonyl-urée (par exemple glibenclamide, glimepiride, glipizide), les thiazoline-diones, les glinides, le DPP-IV inhibiteur (inhibiteur de dipeptidylpeptidase), le GLP-1 (Glucagon-like peptide-1) ou ses analogues.
Ces compositions comprennent également un véhicule ou excipient pharmaceutiquement acceptable.

L'invention concerne également une méthode de traitement de pathologies associées au syndrome d'insulino-résistance (ou syndrome X), notamment du diabète de type II, comprenant l'administration, au patient qui en a besoin, d'une quantité suffisante d'au moins un composé selon l'invention avec un véhicule ou excipient pharmaceutiquement acceptable.

L'identification du patient qui a besoin du traitement ci-dessus indiqué est définie par l'homme du métier. Un vétérinaire ou un médecin peut identifier, par l'intermédiaire de tests cliniques, d'examen physique, de tests ou diagnostics biologiques et par l'historique familial et/ou médical, les sujets qui ont besoin d'un tel traitement.

On entend par quantité suffisante, une quantité de composé selon la présente invention efficace pour prévenir ou traiter des conditions pathologiques. La quantité suffisante peut être déterminée par l'homme du métier, par l'intermédiaire de technique conventionnelle et par l'observation des résultats obtenus dans des circonstances analogues. Pour déterminer la quantité suffisante différents facteurs doivent être pris en compte par l'homme du métier, notamment et sans y être limité : le sujet, sa taille, son âge, son état de santé général, la maladie impliquée et son degré de sévérité ; la réponse du sujet, le type de composé, le mode d'administration, la biodisponibilité de la composition administrée, le dosage, l'utilisation concomitante d'autres médicaments, etc. De préférence, 5 à 500 mg/jour du composé selon l'invention sont administrés au patient en une ou plusieurs prises, de préférence en une prise.
La présente invention concerne également une méthode de traitement de pathologies associées au syndrome d'insulino-résistance (ou syndrome X), notamment du diabète de type II, comprenant l'administration, au patient qui en a besoin, d'une quantité suffisante d'au moins un composé selon l'invention avec un véhicule ou excipient pharmaceutiquement acceptable et d'au moins un autre agent antidiabétique, par exemple choisi parmi l'insuline, par exemple choisi parmi l'insuline, les sulfonyl-urée (par exemple glibenclamide, glimepiride, glipizide), les thiazoline-diones, les glinides, le DPP-IV inhibiteur (inhibiteur de dipeptidylpeptidase), le GLP-1 (Glucagon-like peptide-1) ou ses analogues.

La présente invention va maintenant être décrite à l'aide d'exemples non limitatifs.

### Exemple 1 : Préparation de composés selon l'invention

### Schéma générale de synthèse

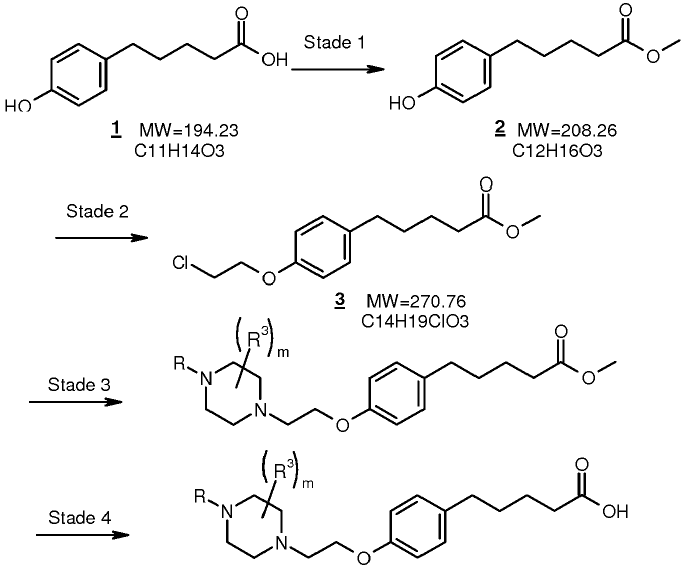

R = R² L'Etape 1 est une réaction d'estérification, les étapes 2 et 3 sont des réactions de substitutions nucléophiles et l'étape 4 est une réaction d'hydrolyse.

### Etape 1 : Estérification

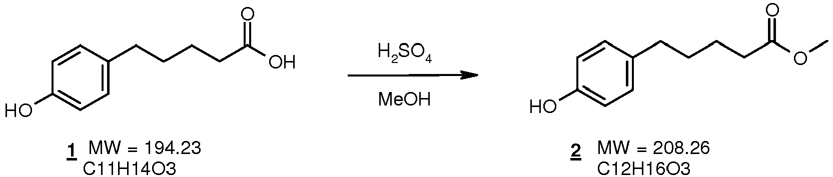

Equipement : Tricol de 500ml équipé d'une agitation magnétique, d'un réfrigérant et placé sous balayage d'azote - Bain d'huile.

L'acide 5-(4-hydroxyphényl)-pentanoique **1** (25g) est mis en solution dans le méthanol (375ml) avant de couler lentement une solution d'acide sulfurique (25ml). La solution ainsi obtenue est chauffée à 65°C pour la nuit.
L'avancement de la réaction est contrôlé par CCM (éluant : Heptane/Acétate d'éthyle : 1/1). Après une nuit d'agitation dans ces conditions, on observe la disparition de l'acide de départ **1** au profit d'un produit moins polaire.
Le milieu réactionnel est concentré à sec sous pression réduite. Le résidu obtenu est repris par du dichlorométhane (300ml). Le milieu hétérogène est neutralisé et basifié, avec précaution, avec une solution saturée d'hydrogénocarbonate de sodium (pH 8-9). La phase aqueuse est ensuite extraite trois fois par du dichlorométhane. Une fois rassemblées, les phases organiques sont lavées une fois par une solution de chlorure de sodium saturée, séchées sur sulfate de magnésium, filtrées puis concentrées sous pression réduite pour fournir une huile rosée (27g).
Rendement 99%

### Etape 2 : Substitution nucléophile

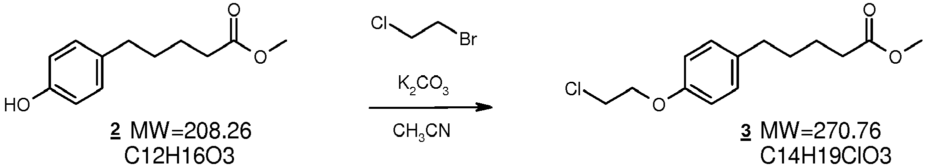

Equipement : Tricol de 500ml équipé d'une agitation magnétique, d'un réfrigérant et placé sous balayage d'azote - Bain d'huile.

L'ester 5-(4-hydroxyphényl)-pentanoate de méthyle **2** (26.8g) est mis en solution dans l'acétonitrile (250ml). Le carbonate de potassium (53.36g), préalablement séché, est ajouté à la solution. Le milieu réactionnel est chauffé à 50°C avant de couler lentement une solution de 1-bromo-2-chloroéthane (55.36g) dans l'acétonitrile (60ml). Le milieu réactionnel est chauffé à 80°C pour la nuit.
L'avancement de la réaction est contrôlé par CCM (éluant : Heptane/Acétate d'éthyle : 7/3). Après 24H d'agitation dans ces conditions, on observe toujours la présence du phénol **2**. On ajoute une quantité supplémentaire de 1-bromo-2-chloroéthane (9.23g, 64.3mmol, 0.5éq.) et on maintient le milieu réactionnel sous agitation à 80°C pendant encore 24H. Un contrôle CCM montre que la réaction n'évolue pas. Une RMN du proton d'un aliquot du milieu réactionnel permet de quantifier les composés **2** et **3** selon un ratio de l'ordre de 33/66. Après retour à température ambiante, le milieu réactionnel est filtré pour éliminer le carbonate de potassium. Le carbonate de potassium est rincé par de l'acétate d'éthyle puis le filtrat est concentré à sec sous pression réduite. Le résidu obtenu est repris par de l'eau (100ml) et la phase aqueuse est extraite trois fois par de l'acétate d'éthyle (100ml). Les phases organiques sont rassemblées, lavées avec une solution d'hydroxyde de sodium 1M (200ml) puis à l'eau (100ml), séchées sur sulfate de magnésium, filtrées puis concentrées sous pression réduite pour fournir une huile beige (39.5g).

L'analyse RMN-1 H du brut réactionnel révèle la présence du dérivé chloré **3** attendu en mélange avec près de 30% de matière première **2**.
Le brut réactionnel est purifié par chromatographie sur gel de silice (1 L). Les composés à séparer sont élués en utilisant un gradient heptane / acétate d'éthyle.
Le composé souhaité est isolé sous la forme d'une huile (22.4g).
Rendement 64%

### Etape 3 : Substitution nucléophile

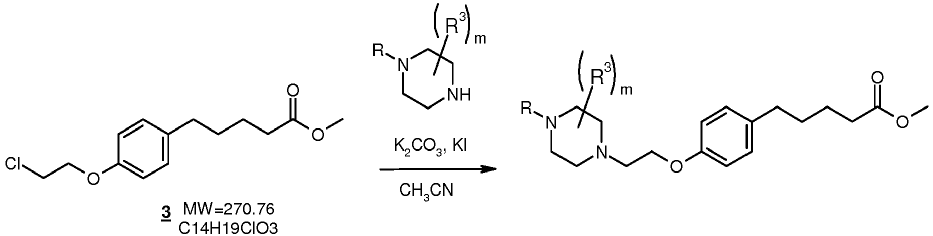

Equipement : Appareil Stem équipé d'un système de chauffage et d'une agitation orbitalaire, Réacteurs de 9ml - Manifold - système d'évaporation multivac - Appareil pour extraction Allexis

Sous balayage d'azote, le dérivé chloré **3** (500mg, 1.85mmol, 1 éq.) est réparti dans les différents réacteurs puis mis en solution dans l'acétonitrile (5ml) en présence de R-pipérazine (1.85mmol, 1 éq.), de carbonate de potassium (766mg, 5.54mmol, 3éq.) préalablement séché et de iodure de potassium (307mg, 1.85mmol, 1 éq.). Après avoir réalisé un balayage d'azote, les réacteurs sont fermés et chauffés à 80°C.
Après 72 heures, le chauffage est arrêté. Après retour à température ambiante, les différents milieux réactionnels sont filtrés en parallèle sur cartouche Supelco reliée à un Manifold pour éliminer les sels inorganiques. Après rinçage par de l'acétonitrile, les filtrats sont concentrés à sec sous pression réduite à l'aide du multivac. Les résidus obtenus sont repris par de l'eau (20ml) et extraits trois fois, en parallèle sur appareil Allexis, par de l'acétate d'éthyle (10ml). Les différentes phases organiques rassemblées sont séchées sur sulfate de magnésium, filtrées puis concentrées sous pression réduite à l'aide du multivac.
Les différents bruts réactionnels sont purifiés par chromatographie sur colonne prépackée Redisep 40g, système Biotage SP4, en utilisant un gradient dichlorométhane / méthanol.

### Etape 4 : Hydrolyse

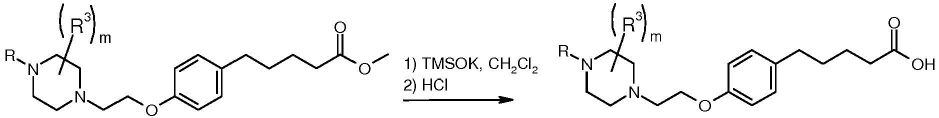

Equipement : Appareil Stem équipé d'un système de chauffage et d'une agitation orbitalaire, Réacteurs de 9ml - Système d'évaporation multivac

Les différents esters issus de **3** sont mis en solution dans le dichlorométhane (5ml) en présence de triméthylsilanoate de potassium. Le milieu réactionnel est chauffé à 35°C pour la nuit.
Après une nuit d'agitation dans ces conditions, un contrôle par CCM (Eluant : dichlorométhane/Méthanol 98/2, révélation UV) permet de vérifier la disparition des esters au profit de produits plus polaires.
Les différents milieux réactionnels sont concentrés à sec sous pression réduite. Les résidus obtenus sont triturés par un mélange de diéthyle éther (4 volumes) et d'éthanol (2 volumes) pour éliminer l'excès de triméthylsilanoate de potassium et le triméthylméthoxysilane résiduel. Après filtration les carboxylates de potassium sont isolés et séchés sous pression réduite.
Chaque carboxylate de potassium est mis en solution dans un minimum d'eau distillée (1 à 11ml) avant d'additionner une solution d'acide chlorhydrique 1N (2éq.). Après 30 minutes d'agitation, les acides formés précipitent sous la forme d'une gomme. Chaque acide est trituré régulièrement jusqu'à l'apparition d'un précipité poudreux. Les précipités sont filtrés, lavés une fois par de l'eau puis séchés sous pression réduite en présence de P₂O₅.

Les composés suivant ont été obtenus en mettant en oeuvre le procédé selon le schéma général ci-dessus.

| Rendement : 85% Structure : | | |
|---|---|---|
| 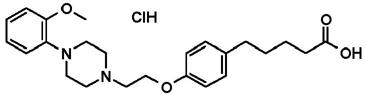 | Composé 1 | |
| | Masse moléculaire : | 448.99 |
| | Formule brute : | C₂₄H₃₃ClN₂O₄ |
| | Forme/couleur : | Solide blanc |

RMN-1H (MeOD, 300MHz, δ ppm) : 1.47-1.59 (m, 4H); 2.23 (t, 2H); 2.53 (t, 2H); 3.19-3.62 (m, 10H); 3.80 (s, 3H); 4.33 (t, 2H); 6.84-7.04 (m, 6H); 7.09 (d, 2H)
MS (ESI) : 413.20 (MH⁺); 411.20 (MH⁻)

| Rendement : 80% Structure : | | |
|---|---|---|
| 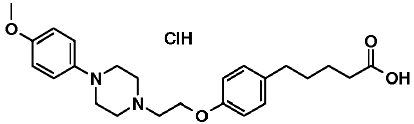 | Composé 2 | |
| | Masse moléculaire : | 448.99 |
| | Formule brute : | C₂₄H₃₃ClN₂O₄ |
| | Forme/couleur : | Solide blanc |

RMN-1 H (MeOD, 300MHz, δ ppm) : 1.60-1.66 (m, 4H); 2.31 (t, 2H); 2.60 (t, 2H); 3.28-3.38 (m, 8H); 3.45 (t, 2H); 3.76 (s, 3H); 4.33 (t, 2H); 6.87-7.02 (m, 6H); 7.16 (d, 2H)
MS (ESI) : 413.20 (MH⁺); 411.20 (MH⁻)

| Rendement : 76% Structure : | | |
|---|---|---|
| 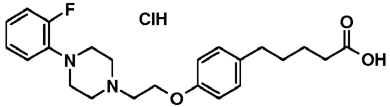 | Composé 3 | |
| | Masse moléculaire : | 436.96 |
| | Formule brute : | C₂₃H₃₀ClFN₂O₃ |
| | Forme/couleur : | Solide blanc |

RMN-1 H (MeOD, 300MHz, δ ppm) : 1.51-1.58 (m, 4H); 2.23 (t, 2H); 2.53 (t, 2H); 3.28-3.38 (m, 4H); 3.47-3.54 (m, 4H); 3.59 (t, 2H); 4.32 (t, 2H); 6.89 (d, 2H); 6.98-7.08 (m, 4H); 7.09 (d, 2H)
MS (ESI) : 401.20 (MH⁺); 399.30 (MH⁻)

| Rendement : 81% Structure : | | |
|---|---|---|
| 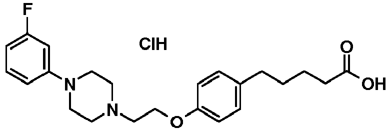 | Composé 4 | |
| | Masse moléculaire : | 436.96 |
| | Formule brute : | C₂₃H₃₀ClFN₂O₃ |
| | Forme/couleur : | Solide blanc |

RMN-1 H (MeOD, 300MHz, δ ppm) : 1.58-1.66 (m, 4H); 2.31 (t, 2H); 2.61 (t, 2H); 3.49-3.59 (m, 8H); 3.67 (t, 2H); 4.40 (t, 2H); 6.65 (td, 1 H); 6.79 (dt, 1 H); 6.84 (dd, 1 H); 6.97 (d, 2H); 7.17 (d, 2H); 7.29 (q, 1 H)
MS (ESI) : 401.20 (MH⁺); 399.30 (MH⁻)

| Rendement : 90% Structure : | | |
|---|---|---|
| | Composé 5 | |
| 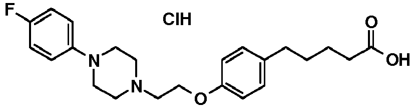 | Masse moléculaire : | 436.96 |
| | Formule brute : | C₂₃H₃₀ClFN₂O₃ |
| | Forme/couleur : | Solide blanc |

RMN-1 H (MeOD, 300MHz, δ ppm) : 1.60-1.66 (m, 4H); 2.31 (t, 2H); 2.61 (t, 2H); 3.39-3.45 (m, 4H); 3.51-3.56 (m, 4H); 3.64 (t, 2H); 4.39 (t, 2H); 6.96 (d, 2H); 7.05 (dd, 4H); 7.17 (d, 2H)
MS (ESI) : 401.10 (MH⁺); 399.20 (MH⁻)

| Rendement : 76% Structure : | | |
|---|---|---|
| | Composé 6 | |
| 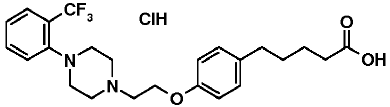 | Masse moléculaire : | 486.97 |
| | Formule brute : | C₂₄H₃₀ClF₃N₂O₃ |
| | Forme/couleur : | Solide blanc |

RMN-1 H (MeOD, 300MHz, δ ppm) : 1.58-1.67 (m, 4H); 2.31 (t, 2H); 2.61 (t, 2H); 3.19-3.24 (m, 4H); 3.35-3.41 (m, 4H); 3.51 (t, 2H); 4.35 (t, 2H); 6.95 (d, 2H); 7.16 (d, 2H); 7.39 (t, 1 H);7.58 (d, 1 H); 7.67 (t, 1 H); 7.71 (d, 1 H)
MS (ESI) : 451.20 (MH⁺); 449.20 (MH⁻)

| Rendement : 57% Structure : | | |
|---|---|---|
| 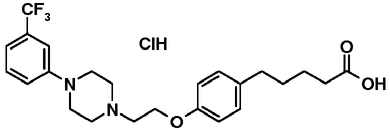 | Composé 7 | |
| | Masse moléculaire : | 486.97 |
| | Formule brute : | C₂₄H₃₀ClF₃N₂O₃ |
| | Forme/couleur : | Solide blanc |

RMN-1 H (MeOD, 300MHz, δ ppm) : 1.60-1.66 (m, 4H); 2.31 (t, 2H); 2.61 (t, 2H); 3.53-3.63 (m, 8H); 3.68 (t, 2H); 4.41 (t, 2H); 6.97 (d, 2H); 7.18 (d, 2H); 7.21 (d, 1 H); 7.30 (dd, 2H); 7.49 (t, 1 H)
MS (ESI) : 451.20 (MH⁺); 449.20 (MH⁻)

| Rendement : 74% Structure : | | |
|---|---|---|
| | Composé 8 | |
| 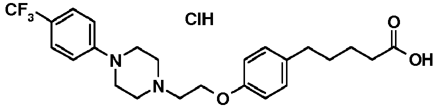 | Masse moléculaire : | 486.97 |
| | Formule brute : | C₂₄H₃₀ClF₃N₂O₃ |
| | Forme/couleur : | Solide blanc |

RMN-1 H (MeOD, 300MHz, δ ppm) : 1.59-1.66 (m, 4H); 2.31 (t, 2H); 2.61 (t, 2H); 3.40-3.46 (m, 4H); 3.51-3.62 (m, 6H); 4.36 (t, 2H); 6.95 (d, 2H); 7.12-7.18 (m, 4H); 7.56 (d, 2H) MS (ESI) : 451.20 [MH⁺]; 449.20 (MH⁻)

| Rendement : 67% Structure : | | |
|---|---|---|
| | Composé 9 | |
| 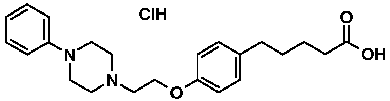 | Masse moléculaire : | 418.97 |
| | Formule brute : | C₂₃H₃₁ClN₂O₃ |
| | Forme/couleur : | Solide blanc |

RMN-1 H (MeOD, 300MHz, δ ppm) : 1.60-1.65 (m, 4H); 2.30 (t, 2H); 2.59 (t, 2H); 3.03-3.07 (m, 4H); 3.14 (t, 2H); 3.29-3.33 (m, 4H); 4.24 (t, 2H); 6.85-6.92 (m, 3H); 7.01 (d, 2H); 7.13 (d, 2H); 7.24-7.29 (m, 2H)
MS (ESI) : 383.20 (MH⁺); 381.20 (MH⁻)

| Rendement : 68% Structure : | | |
|---|---|---|
| | Composé 10 | |
| 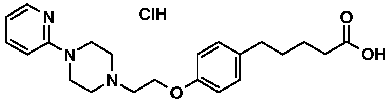 | Masse moléculaire : | 419.96 |
| | Formule brute : | C₂₂H₃₀ClN₃O₃ |
| | Forme/couleur : | Solide blanc |

RMN-1 H (MeOD, 300MHz, δ ppm) : 1.60-1.65 (m, 4H); 2.30 (t, 2H); 2.59 (t, 2H); 2.97-3.01 (m, 4H); 3.12 (t, 2H); 3.64-3.68 (m, 4H); 4.24 (t, 2H); 6.74 (dd, 1 H); 6.88 (dd, 1 H); 6.90 (d, 2H); 7.13 (d, 2H); 7.61 (td, 1 H); 8.13 (dd, 1 H)
MS (ESI) : 384.20 (MH⁺)

| Rendement : 72% Structure : | | |
|---|---|---|
| | Composé 11 | |
| 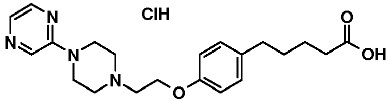 | Masse moléculaire : | 420.94 |
| | Formule brute : | C₂₁H₂₉ClN₄O₃ |
| | Forme/couleur : | Solide blanc |

RMN-1 H (MeOD, 300MHz, δ ppm) : 1.60-1.66 (m, 4H); 2.31 (t, 2H); 2.59 (t, 2H); 2.93-2.97 (m, 4H); 3.08 (t, 2H); 3.74-3.78 (m, 4H); 4.23 (t, 2H); 6.90 (d, 2H); 7.13 (d, 2H); 7.84 (d, 1H); 8.14 (dd, 1H); 8.25 (d, 1H)
MS (ESI) : 385.20 (MH⁺); 383.20 (MH⁻)

| Rendement : 79% Structure : | | |
|---|---|---|
| 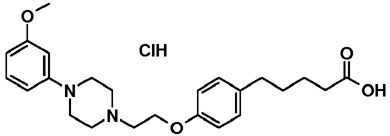 | Composé 12 | |
| | Masse moléculaire : | 448.99 |
| | Formule brute : | C₂₄H₃₃ClN₂O₄ |
| | Forme/couleur : | Solide blanc |

RMN-1 H (MeOD, 300MHz, δ ppm) : 1.60-1.65 (m, 4H); 2.31 (t, 2H); 2.61 (t, 2H); 3.45-3.59 (m, 8H); 3.67 (t, 2H); 3.78 (t, 3H); 4.40 (t, 2H); 6.54 (dd, 1 H); 6.58 (t, 1 H); 6.63 (dd, 1 H); 6.97 (d, 2H); 7.17 (d, 2H); 7.20 (d, 1 H)
MS (ESI) : 413.20 (MH⁺); 411.20 (MH⁻)

### Exemple 2 : Etude de l'activité antidiabétiaue chez la souris db/db

L'activité antidiabétique d'un composé de formule (I) par voie orale sur un modèle animal de diabète de type 2, la souris db/db a été testé.

Les souris diabétiques sont utilisées à l'âge de 8 semaines; elles présentent alors une hyperglycémie très élevée. La stabulation des animaux est réalisée au minimum pendant une semaine après réception et jusqu'au jour de l'expérimentation dans une animalerie à température régulée à 21-22 °C et soumise à un cycle fixe de lumière (de 7 h à 19 h) et d'obscurité (de 19 h à 7 h). Leur alimentation a consisté en un régime d'entretien ; eau et nourriture ont été fournies « ad libitum ».

Les souris sont traitées par voie orale avec le produit à tester (200 mg/kg per os) en suspension dans un mélange d'eau et de méthyl cellulose ou le véhicule (mélange d'eau et de méthyl cellulose) le matin du jour de l'expérimentation. Juste avant l'administration du produit et une et deux heures après l'administration du produit, un prélèvement sanguin (une goutte de sang) est fait suite à une petite incision de la queue pour une détermination de la glycémie.

Cette glycémie est mesurée à l'aide d'un glucomètre (Lifescan OneTouch Ultra, LifeScan, Johnson-Johnson Company) et de bandelettes de mesure de glycémie OneTouch Ultra.

Le tableau 1 rassemble les résultats obtenus. Ces résultats expriment les pourcentages de chute de la glycémie initiale. Pour comparaison, la chute de la glycémie est également rapportée pour des animaux témoins n'ayant reçu que le véhicule et pour la metformine.

**Tableau 1**

| | Chute de la glycémie (en % de la valeur initiale) | |
|---|---|---|
| | Au bout d'1 heure | Au bout de 2 heures |
| Témoin | 5 | 11 |
| Composé 1 | 34 | 44 |
| Metformine | 22 | 27 |

Ces résultats montrent l'efficacité des composés de formule (I) pour induire une diminution de la glycémie chez les animaux diabétiques.

## Revendications

1. Composé de formule (I) 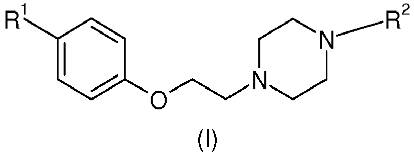 dans laquelle :
▪ R¹ représente un groupe -(CH₂)₄C(O)OH ;
▪ R² représente :
- un groupe phényle non substitué ou substitué par un ou plusieurs substituants choisis parmi :
- un groupe alkoxy en C₁ à C₆, linéaire ou ramifié ;
- un atome d'halogène ;
- un groupe hydrocarboné, linéaire ou ramifié, en C₁ à C₅ ;
- un groupe hydrocarboné, linéaire ou ramifié, en C₁ à C₅, substitué par un ou plusieurs atomes d'halogène ;
- un groupe hétérocycle aromatique en 5 ou 6 membres comprenant 1, 2 ou 3 hétéroatomes, identiques ou différents, choisis parmi azote, oxygène et soufre ;
leurs énantiomères, diastéréoisomères et leurs sels d'addition avec des bases ou des acides pharmaceutiquement acceptables.

2. Composé selon la revendication 1 pour lequel R² représente :
- un phényl, non substitué ou substitué par un ou plusieurs substituants, identiques ou différents, choisi parmi :
• un groupe alkoxy en C₁ à C₆, linéaire ou ramifié ;
• un atome d'halogène ;
• un groupe alkyle en C₁ à C₅, linéaire ou ramifié, substitué par un ou plusieurs atomes d'halogène ;
- un hétéroaryl monocyclique choisi parmi

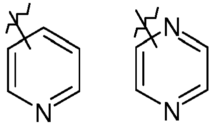

3. Procédé de préparation (P1) d'un composé selon l'une quelconque des revendications 1 ou 2 comprenant :
(a) la protection de la fonction acide d'un composé de formule (II) 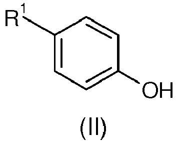 dans laquelle R¹ est tel que défini par les revendications 1 et 2 ;
(b) la réaction du composé obtenu à l'étape (a) avec un composé de formule R⁴-(CH₂)₂-R⁵ dans laquelle R⁴ et R⁵, identiques ou différents représentent un groupe partant, de préférence R⁵ est meilleur nucléofuge que R⁴ ;
(c) la réaction du composé obtenu à l'étape (b) avec un composé de formule (III) 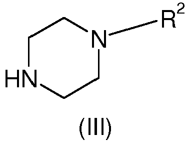 dans laquelle R² est tel que défini par les revendications 1 et 2 ;
(d) déprotection du composé obtenu à l'étape (c).

4. Procédé de préparation (P2) d'un composé selon l'une quelconque des revendications 1 et 2 comprenant :
(i) la réaction d'un composé de formule (III) 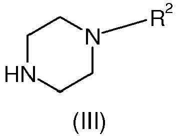 dans laquelle R² est tel que défini par les revendications 1 et 2 avec un composé de formule R⁴-(CH₂)₂-R⁵, dans laquelle R⁴ et R⁵ sont tels que définis à la revendication 3 ;
(ii) la protection de la fonction acide d'un composé de formule (II) 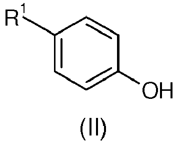 dans laquelle R¹ représente est tel que défini par les revendications 1 et 2;
(iii) la réaction entre le composé obtenu à l'étape (i) et le composé obtenu à l'étape (ii) ;
(iv) la déprotection du composé obtenu à l'étape (iii).

5. Composé selon l'une des revendications 1 ou 2 en tant que médicament.

6. Composé selon l'une des revendications 1 ou 2 pour son utilisation pour le traitement de pathologies associées au syndrome d'insulinorésistance.

7. Composé pour son utilisation selon la revendication 6 pour le traitement du diabète de type (II).

8. Composition pharmaceutique comprenant à titre de principe actif au moins un composé selon l'une des revendications 1 ou 2.

9. Composition pharmaceutique selon la revendication 8, comprenant en outre au moins un autre agent antidiabétique.

## Patentansprüche

1. Verbindung der Formel (I) 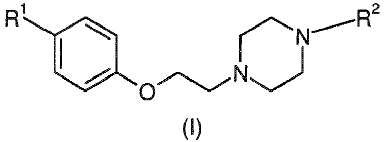 wobei:
▪ R¹ eine -(CH₂)₄C(O)OH Gruppe darstellt;
▪ R²:
- eine Phenylgruppe, unsubstituiert oder substituiert mit einem oder mehreren Substituenten ausgewählt aus:
• einer C₁ bis C₆ Alkoxygruppe, linear oder verzweigt;
• einem Halogenatom;
• einer C₁ bis C₅ Kohlenwasserstoffgruppe, linear oder verzweigt;
• einer C₁ bis C₅ Kohlenwasserstoffgruppe, linear oder verzweigt, substituiert mit einem oder mehreren Halogenatomen;
- eine aromatische Heterozyklusgruppe mit 5 oder 6 Gliedern umfassend 1, 2 oder 3 Heteroatome, gleich oder verschieden, ausgewählt aus Stickstoff, Sauerstoff und Schwefel darstellt;
ihre Enantiomere, Diastereoisomere und ihre Salze der Addition mit pharmazeutisch annehmbaren Basen oder Säuren.

2. Verbindung gemäß Anspruch 1, wobei R²:
- ein Phenyl, unsubstituiert oder substituiert mit einem oder mehreren Substituenten, gleich oder verschieden, ausgewählt aus:
• einer C₁ bis C₆ Alkoxygruppe, linear oder verzweigt;
• einem Halogenatom;
• einer C₁ bis C₅ Alkylgruppe, linear oder verzweigt, substituiert mit einem oder mehreren Halogenatomen;
- ein monozyklisches Heteroaryl ausgewählt aus 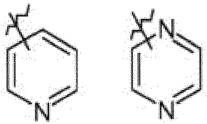 darstellt.

3. Verfahren zur Herstellung (P1) einer Verbindung gemäß einem der Ansprüche 1 oder 2, umfassend:
(a) die Schützung der Säurefunktion einer Verbindung der Formel (II) 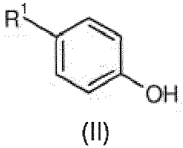 wobei R¹ wie in den Ansprüchen 1 und 2 definiert ist;
(b) die Reaktion der Verbindung erhalten in Schritt (a) mit einer Verbindung der Formel R⁴-(CH₂)₂-R⁵, wobei R⁴ und R⁵, gleich oder verschieden, eine Abgangsgruppe darstellen, vorzugsweise ist R⁵ ein besseres Nukleofug als R⁴;
(c) die Reaktion der Verbindung erhalten in Schritt (b) mit einer Verbindung der Formel (III) 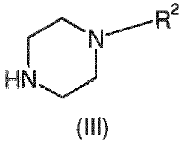 wobei R² wie in den Ansprüchen 1 und 2 definiert ist;
(d) Entschützung der Verbindung erhalten in Schritt (c).

4. Verfahren zur Herstellung (P2) einer Verbindung gemäß einem der Ansprüche 1 und 2, umfassend:
(i) die Reaktion einer Verbindung der Formel (III) 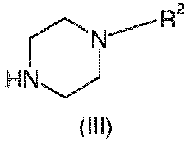 wobei R² wie in den Ansprüchen 1 und 2 definiert ist, mit einer Verbindung der Formel R⁴-(CH₂)₂-R⁵, wobei R⁴ und R⁵ wie in Anspruch 3 definiert sind;
(ii) die Schützung der Säurefunktion einer Verbindung der Formel (II) 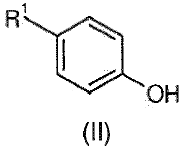 wobei R¹ das wie in den Ansprüchen 1 und 2 definierte darstellt;
(iii) die Reaktion zwischen der Verbindung erhalten in Schritt (i) mit der Verbindung erhalten in Schritt (ii);
(iv) die Entschützung der Verbindung erhalten in Schritt (iii).

5. Verbindung gemäß einem der Ansprüche 1 oder 2 als Medikament.

6. Verbindung gemäß einem der Ansprüche 1 oder 2 für ihre Verwendung zur Behandlung von Erkrankungen, die mit dem Syndrom der Insulinresistenz assoziiert sind.

7. Verbindung für ihre Verwendung gemäß Anspruch 6 für die Behandlung des Diabetes Typ (II).

8. Pharmazeutische Zusammensetzung umfassend als Wirksubstanz mindestens eine Verbindung gemäß einem der Ansprüche 1 oder 2.

9. Pharmazeutische Zusammensetzung gemäß Anspruch 8, umfassend des Weiteren mindestens einen anderen antidiabetischen Wirkstoff.

## Claims

1. Compound of formula (I) 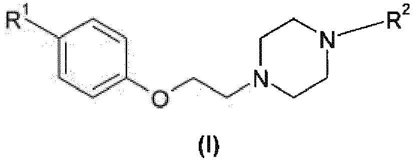 in which:
▪ R¹ represent a -(CH₂)₄C(O)OH group;
▪ R² represents:
- a phenyl group non-substituted or substituted by one or more substituents chosen among:
- a C₁ to C₆ alkoxy group, linear or branched;
- a halogen atom;
- a C₁ to C₅ linear or branched hydrocarbon group;
- a C₁ to C₅ linear or branched hydrocarbon group, substituted by one or more halogen atoms;
- a heterocycle aromatic group with 5 or 6 members, comprising 1, 2 or 3 heteroatoms, identical or different, chosen among nitrogen, oxygen or sulfur;
enantiomers and diastereoisomers thereof and the addition salts thereof with pharmaceutically acceptable bases or acids.

2. Compound according to claim 1, in which R² represents
- a phenyl, non-substituted or substituted by one or more substituents, identical or different, chosen from:
• a C₁ to C₆ alkoxy group, linear or branched;
• a halogen atom;
• a C₁ to C₅ alkyl group, linear or branched, substituted by one or more halogen atoms;
- a monocyclic heteroaryl chosen from:

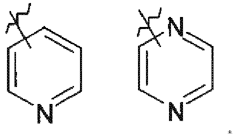

3. Method (P1) for preparing a compound according to any one of claims 1 or 2, comprising:
(a) the protection of the acid function of a compound of formula (II) 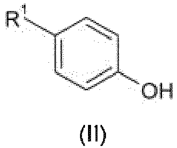 in which R¹ is as defined by claims 1 and 2;
(b) the reaction of the compound obtained at step (a) with a compound of formula R⁴-(CH₂)₂-R⁵ in which R⁴ and R⁵, identical or different, represent a leaving group, and preferably R⁵ is a better nucleofuge than R⁴;
(c) the reaction of the compound obtained at step (b) with a compound of formula (III) 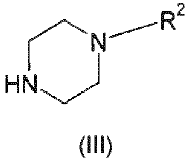 in which R² is as defined by claims 1 and 2;
(d) deprotection of the compound obtained at step (c).

4. Method (P2) for preparing a compound according to any one of claims 1 and 2, comprising:
(i) the reaction of a compound of formula (III) 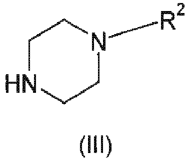 in which R² is as defined by claims 1 and 2 with a compound of formula R⁴-(CH₂)₂-R⁵, in which R⁴ and R⁵ are as defined in claim 3;
(ii) the protection of the acid function of a compound of formula (II) 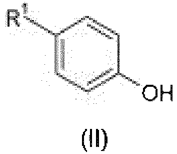 in which R¹ is as defined by claims 1 and 2;
(iii) the reaction between the compound obtained at step (i) and the compound obtained at step (ii);
(iv) deprotection of the compound obtained at step (iii).

5. Compound according to claim 1 or 2 as a medication.

6. Compound according to claim 1 or 2 for use thereof for treating pathologies associated with the insulin-resistance syndrome.

7. Compound for use thereof according to claim 6 for treating type 2 diabetes.

8. Pharmaceutical composition comprising, as active principle, at least one compound according to any one of claims 1 or 2

9. Pharmaceutical composition according to claim 8, also comprising at least one other anti-diabetic agent.
